# EUROPEAN PATENT SPECIFICATION

(11) **EP 3 262 067 B1**
(45) Date of publication and mention of the grant of the patent: **06.02.2019**
(21) Application number: 16705971.6
(22) Date of filing: 24.02.2016
(51) Int. Cl.: C07K 14/50, A61K 38/18, A61K 8/64

(54) **NEW DODECAPEPTIDES AND MICROCAPSULES FUNCTIONALISED WITH THEM TARGETED TO FIBROBLASTS**
NEUE DODEKAPEPTIDE UND MIKROKAPSELN, DIE MIT IHNEN FUNKTIONALISIERT SIND, MIT ABZIELUNG AUF FIBROBLASTEN
NOUVEAUX DODÉCAPEPTIDES ET MICROCAPSULES FONCTIONNALISÉES AVEC CEUX-CI, CIBLÉES SUR LES FIBROBLASTES

(30) Priority: 25.02.2015 EP 15382077
(43) Date of publication of application: 03.01.2018
(73) Proprietor: Infinitec Activos S.L., 08170 Montornés del Vallès, Barcelona (ES)
(72) Inventor: MOURELLE MANCINI, Marisabel, 08028 Barcelona (ES); CRUZ RICONDO, Luis Javier, 08011 Barcelona (ES); CARCELLER MARGELI, Magdalena, 08907 Hospitalet de Llobregat - Barcelona (ES)
(74) Representative: ABG Intellectual Property, S.L.
(86) International application number: PCT/EP2016/053879
(87) International publication number: WO 2016/135203

(56) References cited:
- EP-A2- 1 319 667
- WO-A1-2015/014923
- KR-A- 20110 032 587
- KR-B1- 101 042 712
- US-A1- 2006 217 310

## Description

### FIELD OF THE INVENTION

The present invention relates to the field of pharmacy and cosmetics, in particular, it relates to new dodecapeptides capable of binding to FGF receptors on the fibroblasts thereby promoting the synthesis of collagen type I and/or promoting the synthesis of elastin, to microcapsules targeted to fibroblast comprising said dodecapeptides and to their use to promote the synthesis of collagen type I and/or to promote the synthesis of elastin thereby allowing the prevention and/or treatment of skin aging.

### BACKGROUND

The main effects of skin aging are skin wrinkling, sagging, apparent thinning, appearing of age spots and decrase of elasticity. The differences between young and aged sking are originated at borderline between epidermis and the dermis. Since approximately 45 years of age, there is a gradual thinning of epidermis and dermis.

Fibroblast are the primary cell type in the dermis. The main function of fibroblast is the synthesis and maintenance of the extra cellular matrix (ECM), producing collagen, elastin, proteoglicans, fibronectin, and glycosaminoglycans. They are also involved in wound headling processes, which are key factors for keeping a healthy skin.

Collagen is the most abundant protein in humans as well as in skin and confers durability and resilence to the skin.

Fibroblast are stimulated by various cytoquins. Among them the most important are the Transforming Growth Factor beta (TGT-β) and the Fibroblast Growth Factor (FGF). TGT-β stimulates the producction of collagen and fibronectin, while the FGF stimulates the fibroblast proliferation as well as the synthesis of ECM.

Cutaneous aging occurs through two biologically distinct processes: intrinsic and extrinsic aging. The first is a naturally occurring process that results from slow tissue degeneration. In human dermis, intrinsic aging is characterized by three features: atrophy of the dermis due to loss of collagen, degeneration in the elastic fiber network, and loss of hydration. These three features often manifest themselves by a number of sympthoms comprising, among others, decline of skin elasticity, rough skin, skin wrinkling, sagging, apparent skin thinning, and pigmentation. In contrast to intrinsic aging, extrinsic aging is due to environmental factors such as reactive oxygen species (ROS) and stress, which generates rough wrinkles in the skin surface and significantly decreases skin elasticity (Arch Dermatol., 130: 87-95, 1994; J Drugs Dermatol. 2008 Feb;7(2 Suppl):s12-6).

Thus, the promotion of collagen and/or eleastin may help prevent or revert the aging process (Baumann, L. and S. Saghari, Basic Science of the Dermis, in Cosmetic Dermatology: Principles and Practice, Second Edition, L. Baumann, Editor. 2009, McGraw-Hill).

### BRIEF DESCRIPTION OF THE INVENTION

The inventors have surprisingly found that the peptide of formula (I):
R²-Lys¹-Phe²-Asn³-Leu⁴-Pro⁵-Leu⁶-Gly-Asn⁸-Tyr⁹-Lys¹⁰-Lys¹¹-Pro¹²-R¹ (I) (R²-SEQ ID NO: 1-R¹)
or analogues thereof in which one of the aminoacid rests 2 to 12 is replaced with an alanine rest wherein R¹ is selected from the group consisting of -OH, -OR³, -SR³, - NR³R⁴, -OR⁵ and -NHR⁵; R² is selected from the group consisting of H-, R³-C(O)- and R³-OC(O)-, R³ and R⁴ are independently selected from H, C₁-C₂₄ alkyl, C₂-C₂₄ alkenyl and C₆-C₁₀ aryl wherein R⁵ is selected from the group consisting of -(CH₂)₃-PEG-CH₂-NH₂, -PEG-R⁶, - (CH₂)ₙ-O-PEG-R⁶, -CO-O-PEG-R⁶, -CO- (CH₂CH₂ )-CO-O-PEG-R⁶, -CO-(CH₂)ₘ-O-PEG-R⁶, -CO- (CH₂)ₙ-CH(R⁷) -O-PEG-R⁶, -CO- (CH₂)ₘ -CH(R⁷)-O-CO-CH(R⁷)-(CH₂)ₘ-O-PEG-R⁶ and -(1,3,5-triazine)-(O-PEG-R⁶)₂,
PEG is a polyether group selected from the group consisting of
-(CH₂-CH₂-O)ₙ-, -(CH₂-CH(CH₃)-O)ₒ-, -(CH(CH₃)-CH₂-O)ₒ-, -(CH₂-CH₂-O)ₙ-(CH₂-CH(CH₃)-O)ₒ-(CH₂-CH₂-O)ₚ-, -(CH₂-CH₂-O)ₙ-(CH(CH₃)-CH₂-O)ₒ-(CH₂-CH₂-O)ₚ-, -(CH₂-CH₂-O)ₙ-(CH(CH₃)-CH₂-O)ₒ-, -(CH₂-CH₂-O)ₙ- (CH₂-CH(CH₃)-O)ₒ-, -(CH₂-CH(CH₃)-O)ₒ-(CH₂-CH₂-O)ₙ-, -(CH(CH₃)-CH₂-O)ₒ-(CH₂-CH₂-O)ₙ-, -(CH₂-CH( CH₃)-O)ₒ-(CH₂-CH₂-O)ₙ-(CH₂-CH(CH₃)-O)_{q}-, -(CH₂-CH(CH₃)-O)ₒ-(CH₂-CH₂-O)ₙ-(CH(CH ₃)-CH₂-O)_{q}-, -(CH(CH₃)-CH₂O)ₒ-(CH₂-CH₂-O)ₙ-(CH₂-CH(CH₃)-O)_{q}- and -(CH(CH₃)-CH₂O )ₒ-(CH₂-CH₂-O)ₙ-(CH(CH₃)-CH₂-O)ₒ-,
R⁶ is a hydrogen atom or a C₁₋₄ alkyl group; R⁷ is a C₁₋₄ alkyl group, m is an integer from 1 to 4, n and p are integers between 1 and 10 and m and o are integers between 1 and 10 and cosmetically acceptable salts and solvates thereof, are capable of selectively binding to FGF receptors on the fibroblasts thereby promoting the synthesis of collagen type I and/or promoting the synthesis of elastin.

The inventors have also surprisingly found that coupling the peptide of formula (I):
R²-Lys¹-Phe²-Asn³-Leu⁴-Pro⁵-Leu⁶-Gly-Asn⁸-Tyr⁹-Lys¹⁰-Lys¹¹-Pro¹²-R¹ (I) (R²-SEQ ID NO: 1-R¹)
or analogues thereof in which one of the aminoacid rests 2 to 12 is replaced with an alanine rest wherein R¹ is selected from the group consisting of -OH, -OR³, -SR³, - NR³R⁴, -OR⁵ and -NHR⁵, R² is selected from the group consisting of H-, R³-C(O)- and R³-OC(O)-, R³ and R⁴ are independently selected from H, C₁-C₂₄ alkyl, C₂-C₂₄ alkenyl and C₆-C₁₀ aryl,
wherein R⁵ is selected from the group consisting
of -(CH₂)₃-PEG-CH₂-NH₂, -PEG-R⁶, - (CH₂)ₙ-O-PEG-R⁶, -CO-O-PEG-R⁶, -CO-(CH₂CH₂ )-CO-O-PEG-R⁶, -CO-(CH₂)ₘ-O-PEG-R⁶, -CO-(CH₂)ₙ-CH(R⁷) -O-PEG-R⁶, -CO-(CH₂)ₘ -CH(R⁷)-O-CO-CH(R⁷)-(CH₂)ₘ-O-PEG-R⁶ and -(1,3,5-triazine)-(O-PEG-R⁶)₂,
PEG is a polyether group selected from the group consisting of
-(CH₂-CH₂-O)ₙ-, -(CH₂-CH(CH₃)-O)ₒ-, -(CH(CH₃)-CH₂-O)ₒ-, -(CH₂-CH₂-O)ₙ-(CH₂-CH(CH₃)-O)ₒ-(CH₂-CH₂-O)ₚ-, -(CH₂-CH₂-O)ₙ-(CH(CH₃)-CH₂-O)ₒ-(CH₂-CH₂-O)ₚ-, -(CH₂-CH₂-O)ₙ-(CH(CH₃)-CH₂-O)ₒ-, -(CH₂-CH₂-O)ₙ-(CH₂-CH(CH₃)-O)ₒ-, -(CH₂-CH(CH₃)-O)ₒ-(CH₂-CH₂-O)ₙ-, -(CH(CH₃)-CH₂-O)ₒ-(CH₂-CH₂-O)ₙ-, -(CH₂-CH( CH₃)-O)ₒ-(CH₂-CH₂-O)ₙ-(CH₂-CH(CH₃)-O)_{q}-, -(CH₂-CH(CH₃)-O)ₒ-(CH₂-CH₂-O)ₙ-(CH(CH ₃)-CH₂-O)_{q}-, -(CH(CH₃)-CH₂O)ₒ-(CH₂-CH₂-O)ₙ-(CH₂-CH(CH₃)-O)_{q}- and -(CH(CH₃)-CH₂O )ₒ-(CH₂-CH₂-O)ₙ-(CH(CH₃)-CH₂-O)ₒ-,
R⁶ is a hydrogen atom or a C₁₋₄ alkyl group; R⁷ is a C₁₋₄ alkyl group, m is an integer from 1 to 4, n and p are integers between 1 and 10 and m and o are integers between 1 and 10; and cosmetically acceptable salts and solvates thereof, to the outer surface of microcapsules said targeted microcapsules are preferentially bound to fibroblasts and this is a useful property for the selective delivery of active ingredients which may be contained in the microcapsules to the vicinity of fibroblasts. This targeted delivery of active ingredients may be of particular relevance for the delivery of antiaging compounds to fibroblast thereby improving the anti-aging effect of said antiaging compounds.

In a first aspect the present invention relates to a peptide of formula (I)
R²-Lys¹-Phe²-Asn³-Leu⁴-Pro⁵-Leu⁶-Gly-Asn⁸-Tyr⁹-Lys¹⁰-Lys¹¹-Pro¹²-R¹ (I) (R²-SEQ ID NO: 1-R¹)
or analogues thereof in which one of the aminoacid rests 2 to 12 is replaced with an alanine rest, wherein R¹ is selected from the group consisting of -OH, -OR³, -SR³, - NR³R⁴, -OR⁵ and -NHR⁵; R² is selected from the group consisting of H-, R³-C(O)- and R³-OC(O)-, R³ and R⁴ are independently selected from H, C₁-C₂₄ alkyl, C₂-C₂₄ alkenyl and C₆-C₁₀ aryl; wherein R⁵ is selected from the group consisting
of -(CH₂)₃-PEG-CH₂-NH₂, -PEG-R⁶, - (CH₂)ₙ-O-PEG-R⁶, -CO-O-PEG-R⁶, -CO-(CH₂CH₂ )-CO-O-PEG-R⁶, -CO-(CH₂)ₘ-O-PEG-R⁶, -CO-(CH₂)ₙ-CH(R⁷)-O-PEG-R⁶, -CO-(CH₂)ₘ -CH(R⁷)-O-CO-CH(R⁷)-(CH₂)ₘ-O-PEG-R⁶ and -(1,3,5-triazine)-(O-PEG-R⁶)₂,
PEG is a polyether group selected from the group consisting of
-(CH₂-CH₂-O)ₙ-, -(CH₂-CH(CH₃)-O)ₒ-, -(CH(CH₃)-CH₂-O)ₒ-, -(CH₂-CH₂-O)ₙ-(CH₂-CH(CH₃)-O)ₒ-(CH₂-CH₂-O)ₚ-, -(CH₂-CH₂-O)ₙ-(CH(CH₃)-CH₂-O)ₒ-(CH₂-CH₂-O)ₚ-, -(CH₂-CH₂-O)ₙ-(CH(CH₃)-CH₂-O)ₒ-, -(CH₂-CH₂-O)ₙ-(CH₂-CH(CH₃)-O)ₒ-, -(CH₂-CH(CH₃)-O)ₒ-(CH₂-CH₂-O)ₙ-, -(CH(CH₃)-CH₂-O)ₒ-(CH₂-CH₂-O)ₙ-, -(CH₂-CH( CH₃)-O)ₒ-(CH₂-CH₂-O)ₙ-(CH₂-CH(CH₃)-O)_{q}-, -(CH₂-CH(CH₃)-O)ₒ-(CH₂-CH₂-O)ₙ-(CH(CH ₃)-CH₂-O)_{q}-, -(CH(CH₃)-CH₂O)ₒ-(CH₂-CH₂-O)ₙ-(CH₂-CH(CH₃)-O)_{q}- and -(CH(CH₃)-CH₂O )ₒ-(CH₂-CH₂-O)ₙ-(CH(CH₃)-CH₂-O)ₒ-,
R⁶ is a hydrogen atom or a C₁₋₄ alkyl group; R⁷ is a C₁₋₄ alkyl group, m is an integer from 1 to 4, n and p are integers between 1 and 10 and m and o are integers between 1 and 10; and cosmetically acceptable salts and solvates thereof

In a second aspect the present invention provides microcapsules comprising a peptide as defined in the first aspect of the invention coupled to the outer surface of the microcapsules.

In a third aspect the present invention provides a process for obtention of the microcapsules as defined in the second aspect comprising the steps of:
a) forming the microcapsules encapsulating the antiaging compound and
b) coupling a peptide as defined in the first aspect of the invention to the outer surface of the microcapsule after forming the microcapsule.

In a fourth aspect the present invention provides a cosmetic composition comprising the microcapsule as defined in the second aspect.

In a fifth aspect the present invention provides the cosmetic use of a peptide as defined in the first aspect for promoting the synthesis of collagen type I and or the synthesis of elastin.

In a sixth aspect the present invention provides the use of the microcapsules as defined in the second aspect for the cosmetic prevention and/or cosmetic treatment of sking aging.

In a seventh aspect the present invention provides the use of the microcapsules as defined in the second aspect to improve skin aspect by achieving one or more of the following effects: increasing luminosity of skin, increasing skin elasticity, diminishing skin roughness, reducing skin wrinkles, increasing apparent skin thinning, reducing skin pigmentation defaults.

### DETAILED DESCRIPTION OF THE INVENTION

In the context of the present invention, the following terms have the meaning detailed below.

As used in the context of the present invention the term "microcapsule" refers to a particle consisting of a shell comprising a wall-forming material and an encapsulated core located within the shell and having a size ranging from 10 to 10000 nm. In one embodiment the microcapsules have a size (as determined by Scanning Electron Microscopy (SEM)) ranging from 50 to 5000 nm, preferably from 100 to 1000 nm, more preferably from 150 to 450 nm and most preferably from 180 to 400 nm. This size allows the microcapsule to be uptaken by the cells, and to release the actives into the cytosol. In a particular embodiment the average size of the microcapsules is 220 nm.

In this description the abbreviations used for amino acids follow the recommendations of the 1983 IUPAC-IUB Commission of Biochemical Nomenclature specified in Eur. J. Biochem., (1984), 138, 937.

Thus, for example, Arg or R represents NH₂-CH(CH₂-CH₂-CH₂-NH-C(=NH)NH₂)-COOH, Arg- or R- represents NH₂-CH(CH₂-CH₂-CH₂-NH-C(=NH)NH₂)-CO-, -Arg or -R represents -NH-CH(CH₂-CH₂-CH₂-NH-C(=NH)NH₂)-COOH, and -Arg- or -R- represents -NH-CH(CH₂-CH₂-CH₂-NH-C(=NH)NH₂)-CO-. Therefore, the hyphen, which represents the peptide bond, eliminates the OH in the 1 carboxyl group of the amino acid (represented here in the conventional non-ionized form) when situated to the right of the symbol, and eliminates the H of the 2 amino group of the amino acid when situated to the left of the symbol; both modifications can be applied to the same symbol (see Table 1).

The aminoacids are named using the conventional nomenclature in one and three letter codes, as follows:
- alanine, Ala o A,
- phenylalanine, Phe o F,
- lysine, Lys o K,
- glutamine, Gln o Q,
- arginine, Arg o R.

In one embodiment of the present invention the microcapsules according to the second aspect (i.e. microcapsules which comprise a peptide as defined in the first aspect coupled to the outer surface thereof) comprise and antiaging agent in the microcapsule's core. By incorporating the antiaging in the core of the microcapsules its anti-aging effect is improved.

The microcapsules of the invention have the advantage that they reach deep into the hair follicles, where the barrier possess only a few layers of differentiated corneocytes and can be considered highly permeable. Additionally, the hair follicles can act as long-term reservoir, beneficial condition when transdermal delivery is intended. The hair follicle delivery has several pharmacokinetic advantages as a reduction or bypass of the tortuous pathway of the transepidermal absorption, decrease of the drug systemic toxicity when the follicle acts as long term delivery reservoir and increasing additionally the therapeutic index of some drugs as well as reducing the applied dose or frequency of administration.

In an embodiment of the present invention the microcapsule of the invention have at least one shell comprising a polymeric material. In a preferred embodiment the microcapsules are bilayered polymeric capsules, i.e. the microcapsules consists of an inner microcapsule comprising a core and a first polymeric shell which is coated with a second polymeric shell. In a preferred embodiment the polymeric shell comprises one or more biodegradable polymer.

The following dodecapeptides are specific embodiments of the present invention:
- R²-Lys-Phe-Asn-Leu-Pro-Leu-Gly-Asn-Tyr-Lys-Lys-Pro-R¹ (R²-SEQ ID NO: 1-R¹)
- R²-Lys-Ala-Asn-Leu-Pro-Leu-Gly-Asn-Tyr-Lys-Lys-Pro-R¹ (R²-SEQ ID NO: 2-R¹)
- R²-Lys-Phe-Ala-Leu-Pro-Leu-Gly-Asn-Tyr-Lys-Lys-Pro-R¹ (R²-SEQ ID NO: 3-R¹)
- R²-Lys-Phe-Asn-Ala-Pro-Leu-Gly-Asn-Tyr-Lys-Lys-Pro-R¹ (R²-SEQ ID NO: 4-R¹)
- R²-Lys-Phe-Asn-Leu-Ala-Leu-Gly-Asn-Tyr-Lys-Lys-Pro-R¹ (R²-SEQ ID NO: 5-R¹)
- R²-Lys-Phe-Asn-Leu-Pro-Ala-Gly-Asn-Tyr-Lys-Lys-Pro-R¹ (R²-SEQ ID NO: 6-R¹)
- R²-Lys-Phe-Asn-Leu-Pro-Leu-Ala-Asn-Tyr-Lys-Lys-Pro-R¹ (R²-SEQ ID NO: 7-R¹)
- R²-Lys-Phe-Asn-Leu-Pro-Leu-Gly-Ala-Tyr-Lys-Lys-Pro-R¹ (R²-SEQ ID NO: 8-R¹)
- R²-Lys-Phe-Asn-Leu-Pro-Leu-Gly-Asn-Ala-Lys-Lys-Pro-R¹ (R²-SEQ ID NO: 9-R¹)
- R²-Lys-Phe-Asn-Leu-Pro-Leu-Gly-Asn-Tyr-Ala-Lys-Pro-R¹ (R²-SEQ ID NO: 10-R¹)
- R²-Lys-Phe-Asn-Leu-Pro-Leu-Gly-Asn-Tyr-Lys-Ala-Pro-R¹ (R²-SEQ ID NO: 11-R¹)
- R²-Lys-Phe-Asn-Leu-Pro-Leu-Gly-Asn-Tyr-Lys-Lys-Ala-R¹ (R²-SEQ ID NO: 12-R¹)

In an embodiment of the present invention R² is selected from the group consisting of H, C₁₀-C₂₄ alkyloyl and C₁₀-C₂₄ alkenyloyl; more preferably from the group consisting of caproyl (CH₃-(CH₂)₃-C(O)-), lauroyl (CH₃-(CH₂)₁₀-C(O)-), myristoyl (CH₃-(CH₂)₁₂-C(O)-), palmitoyl (CH₃-(CH₂)₁₄-C(O)-), stearoyl (CH₃-(CH₂)₁₆-C(O)-), arachidoyl (CH₃-(CH₂)₁₈-C(O)-) and behenoyl (CH₃-(CH₂)₂₀-C(O)-); still more preferably, R² is palmitoyl.

In embodiment of the present invention R¹ is selected from the group consisting of - OR³, -SR³, -NR³R⁴, -OR⁵ and -NHR⁵ wherein R³ and R⁴ are independently selected from the group consisting of H and C₁-C₁₈ alkyl and wherein R⁵ is a rest of formula - (CH₂)₃-(OCH₂CH₂)ₙ-CH₂-NH₂ wherein n is an integer from 1 to 18; more preferably R¹ is selected from the group consisting of -OH, -SH, -NH₂ and a rest of formula -NH-(CH₂)₃-(OCH₂CH₂)ₙ-CH₂-NH₂ wherein n is an integer from 1 to 6; still more preferably R¹ is NH₂ or a rest of formula -NH-(CH₂)₃-(OCH₂CH₂)₃-CH₂-NH₂.

In a preferred embodiment, R² is palmitoyl and R¹ is -NH₂, i.e., the compound of formula (I) is a peptide having the sequence Palm-Lys-Phe-Asn-Leu-Pro-Leu-Gly-Asn-Tyr-Lys-Lys-Pro-NH₂ (Palm-SEQ ID NO: 1-NH₂) and analogues thereof in which one of the aminoacid rests 2 to 12 is replaced with an alanine rest.

The targeted microcapsules, and more particularly the targeted bilayered microcapsules, can be used as a highly efficient delivery system with sustained and controlled release of the encapsulated active ingredient at the target cells: fibroblasts and/or keratinocytes. In particular, the microcapsules escape from the endosomal and lysosomal compartment so that the encapsulated active ingredient is released directly in the cytoplasm and reaches the target quickly, optimizing the application and the bioavailability of the encapsulated active agents.

In addition, the targeted microcapsules of the invention have the advantage that they show higher penetration into the skin when compared with conventional microcapsules.

Brief, the use of the targeted microcapsules of the present invention allows decreasing the amount needed of the active ingredients with the consequence that potential side-effects are also reduced. Further, the microcapsules have also the advantage that are capable of penetrating skin and show a uniformly distribution on the entire epidermis.

In one embodiment, the polymers forming the microcapsules of the invention are selected from the group consisting of poly(D,L-lactide-co-glycolide), polylactic acids, poly(propylene fumarate-co-ethylene glycol) [P(PF-co-EG)] block copolymer, poly-anhydride poly(fumaric-co-sebacic) anhydride, poly (ethylene oxide)-poly(lactide/glycolide), poly(amino acid), polyvinyl alcohol, alginate, dextran, chitosan, hydroxyapatite, collagen, fibrin, hyaluronic acid, carbomers, poly(amino acid) and poly(ethylene glycol).

In one embodiment, the microcapsules of the invention are bilayered polymeric microcapsules which comprise an "inner layer polymer", and an outer layer polymer in the outer surface of the microcapsule". The inner layer polymers and the shell polymers are selected from the group consisting of poly(D,L-lactide-co-glycolide), polylactic acids, poly(propylene fumarate-co-ethylene glycol) [P(PF-co-EG)] block copolymer, poly-anhydride poly(fumaric-co-sebacic) anhydride, poly (ethylene oxide)-poly(lactide/glycolide), poly(amino acid), polyvinyl alcohol, alginate, dextran, chitosan, hydroxyapatite, collagen, fibrin, hyaluronic acid, carbomers, poly(amino acid) and poly(ethylene glycol). In a preferred embodiment the inner layer polymer and the outer layer polymer are different. In a preferred embodiment, the inner layer polymer is poly (D,L lactide-co-glycolide) (PLGA) and the outer layer polymer is polyvinyl alcohol (PVA). In a preferred embodiment, at least some of the functional groups of the inner layer polymer, preferably when the functional groups are carboxyl groups of the inner layer polymer, are present in the outer surface of the microcapsule, together with the polymer in the outer surface of the microcapsule. In a more preferred embodiment when the inner layer polymer is poly (D,L lactide-co-glycolide) (PLGA) and the outer layer polymer is polyvinyl alcohol (PVA), the carboxyl groups from the PLGA polymer are present on the outside surface of the microcapsule. In a more preferred embodiment, PLGA has a lactide/glycolide molar ratio from 40:60 to 60:40, more preferably 50:50.

In one embodiment the peptide of formula (I) or analogues thereof in which one of the aminoacid rests 2 to 12 is replaced with an alanine rest are attached to the outer layer polymer on the surface of the microcapsules. In a more particular embodiment, the peptide of formula (I) or analogues thereof in which one of the aminoacid rests 2 to 12 is replaced with an alanine rest are attached to the outer layer polymer by a covalent bond. In a preferred embodiment the covalent bond is an amide bond between the amino group of the peptide's N-terminal group and the carboxyl group from the PLGA polymer present outside of the surface. Preferably, the outer layer polymer is polyvinyl alcohol.

The term "alkanoyl", and refers to a radical having the general formula RCO-wherein R is an alkyl group.

The term "alkenyloyl" is the same as alkenoyl, and refers to a radical having the general formula RCO- wherein R is a alkenyl group.

As used herein, the term "alkyl" refers to a linear or branched hydrocarbon chain radical consisting of carbon and hydrogen atoms, containing no unsaturation, which is attached to the rest of the molecule by a single bond and, unless otherwise specified, an alkyl radical typically has 1 to about 24 carbon atoms. One more preferred class of alkyl groups has from 1 to about 16 carbon atoms and a more preferred class of alkyl groups has from 1 to 6 and 14 to 18. Even more preferred are alkyl groups having 1, 2, 3, 4, 15, 16 or 17 carbon atoms. Methyl, ethyl, n-propyl, isopropyl and butyl, including n-butyl, tert-butyl, sec-butyl, isobutyl, pentadecyl, hexadecyl, heptadecyl are particularly preferred alkyl groups in the compounds of the present invention. Another preferred class of alkyl groups has from 6 to about 10 carbon atoms; and even more preferably 7, 8 or 9 carbon atoms. Heptyl, octyl and nonyl are the most preferred alkyl groups of this class.

The term "alkenyl" means a linear or branched hydrocarbon chain radical having one or more carbon-carbon double bonds therein and having from two to twelve carbon atoms, and which is attached to the rest of the molecule by a single bond. The double bond of an alkenyl group can be unconjugated or conjugated to another unsaturated group. Suitable alkenyl groups include, but are not limited to alkenyl groups such as vinyl, allyl, butenyl (e.g. 1-butenyl, 2-butenyl, 3-butenyl), pentenyl (e.g. 1-pentenyl, 2-pentenyl, 3-pentenyl, 4-pentenyl,), hexenyl (e.g. 1-hexenyl, 2-hexenyl, 3-hexenyl, 4-hexenyl, 5-hexenyl,), butadienyl, pentadienyl (e.g. 1,3-pentadienyl, 2,4-pentadienyl), hexadienyl (e.g. 1,3-hexadienyl, 1,4-hexadienyl, 1,5-hexadienyl, 2,4-hexadienyl, 2,5-hexadienyl), 2-ethylhexenyl (e.g. 2-ethylhex-1-enyl, 2-ethylhex-2-enyl, 2-ethylhex-3-enyl, 2-ethylhex-4-enyl, 2-ethylhex-5-enyl,), 2-propyl-2-butenyl, 4,6-dimethyl-oct-6-enyl. An alkenyl group can be unsubstituted or substituted with one or two suitable substituents.

"Aryl" herein refers to single and double ring radicals from 6 to about 10 carbon ring atoms, such as phenyl, naphthyl or indenyl. The aryl radical may be optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, etc.

The peptides of the invention may be prepared by routine techniques of peptide synthesis known to the skilled in the art. One particularly useful technique consists of five steps carried out in a cyclic fashion.
Step 1 - Attaching an amino acid to the polymer The amino acid is reacted with a molecule known as a "linkage agent" that enables it to attach to a solid polymer, and the other end of the linkage agent is reacted with the polymer support.
Step 2 - Protection
   An amino acid is an acid with a basic group at one end and an acid group at the other. To prevent an amino acid from reacting with itself, one of these groups is reacted with something else to make it unreactive.
Step 3 - Coupling
   The protected amino acid is then reacted with the amino acid attached to the polymer to begin building the peptide chain.
Step 4 - Deprotection
   The protection group is now removed from the acid at the end of the chain so it can react with the next acid to be added on. The new acid is then protected (Step 2) and the cycle continues until a chain of the required length has been synthesised.
Step 5 - Polymer removal
   Once the desired peptide has been obtained the bond between the first amino acid and the linkage agent is broken to give the free peptide.

The terminal groups R¹ and R² are introduced by routine techniques known to the skilled in the art.

The cosmetically acceptable salts of the peptides provided by this invention are also within the scope of the present invention. The term "cosmetically acceptable salts" means a salt generally admitted for its use in animals and more particularly in human beings, and includes the salts used to form base addition salts, either inorganic base addition salts, such as for example and in a non-limiting sense, lithium, sodium, potassium, calcium, magnesium or aluminium among others, or organic base addition salts, such as for example and in a non-limiting sense ethylamine, diethylamine, ethylenediamine, ethanolamine, diethanolamine, arginine, lysine, histidine or piperazine among others, or acid addition salts, either organic acid addition salts, such as for example and in a non-limiting sense acetate, citrate, lactate, malonate, maleate, tartrate, fumarate, benzoate, aspartate, glutamate, succinate, oleate, trifluoroacetate, oxalate, pamoate or gluconate among others, or inorganic acid addition salts, such as for example and in a non-limiting sense chloride, sulfate, borate or carbonate among others. The nature of the salt is not critical, provided that it is cosmetically acceptable. The cosmetically acceptable salts of the peptide derivatives of the invention can be obtained by conventional methods well known in the state of the art [Berge S.M., Bighley L.D. and Monkhouse D.C. (1977) "Pharmaceutical Salts" J. Pharm. Sci. 66:1-19].

Another aspect of the present invention relates to a process for the preparation of microcapsules of the invention, comprising the steps of:
a) forming the microcapsules encapsulating the anti-aging compound and
b) coupling a compound of formula (I) of the invention or analogues thereof in which one of the aminoacid rests 2 to 12 is replaced with an alanine rest to the outer surface of the microcapsules before or after forming the microcapsules.

In a preferred embodiment the step of coupling a compound of formula (I) of the invention or analogues thereof in which one of the aminoacid rests 2 to 12 is replaced with an alanine rest, to the outer surface of the microcapsules is done once the microcapsules is formed, i.e. after forming the microcapsules.

In a preferred embodiment, wherein the microcapsules are polymeric bilayered microcapsules, the step of forming the microcapsules encapsulating the anti-aging compound comprises the steps of
a1) mixing the inner layer polymer with the anti-aging compound in a suitable solvent,
a2) emulsifying the mixture obtained in step a) with the outer layer polymer in a suitable solvent, and optionally
a3) isolating the microcapsules.

In one embodiment wherein the microcapsules are polymeric bilayered microcapsules the process of coupling the compound of formula (I) of the invention or analogues thereof in which one of the aminoacid rests 2 to 12 is replaced with an alanine rest, to the outer layer polymer, when said polymer has carboxyl groups and the coupling in step b) comprises the steps of
b1) activating the carboxyl groups on the surface of the microcapsules, and
b2) reacting the activated microcapsules with the N-terminal amine group of the compound of formula (I).

In another embodiment the outer layer polymer has amino groups and the coupling in step b) comprises the steps of
b1) activating the amino groups on the surface of the microcapsules, and
b2) reacting the activated microcapsules with the C-terminal group of the compound of formula (I).

In another embodiment the outer layer polymer has maleimide groups and the coupling in step b) comprises the steps of
b1) activating the maleimide groups on the surface of the microcapsules,
b3) functionalizing the compound of formula (I) with a thiol group by adding an extra amino acid with such group or by modifying at least one of the functional groups of the present amino acids, and
b2) reacting the activated microcapsules with the thiol group of the compound of formula (I).

In one embodiment the suitable solvent for mixing the inner layer polymer with the anti-aging compound is selected from the group consisting of acetone, acetonitrile, dichloromethane (DCM), ethanol, methanol, chloroform, dimethylformamide (DMF), and ethylacetate, preferably acetone.

In one embodiment the suitable solvent for emulsifying the mixture obtained in step a1) with the outer layer polymer is selected from the group consisting of water, acetonitrile, dichloromethane (DCM), ethanol, methanol, chloroform, dimethylformamide (DMF), dimethylsulfoxide (DMSO) and ethylacetate, preferably water, ethanol, methanol, dimethylformamide, and dimethylsulfoxide (DMSO) and more preferably water.

Another aspect of the invention refers to a process for obtention of bilayered microcapsules as defined in the invention comprising the steps of:
i) coupling a compound of formula (I) of the invention to the polymer which serves as outer layer polymer,
ii) mixing the inner layer polymer with the anti-aging compound in a suitable solvent,
iii) emulsifying the polymer obtained in step i) with the mixture obtained in step ii) in a suitable solvent, and optionally
iv) isolating the microcapsules.

In a preferred embodiment the cosmetic composition comprising microcapsules of the invention comprises at least one cosmetically acceptable excipient or adjuvant.

The term excipients or adjuvants also relate to carriers. Such pharmaceutical excipients, adjuvants or carriers can be liquids, such as water, oils or surfactants, including those of petroleum, animal, plant or synthetic origin, such as for example and in a non-limiting sense peanut oil, soybean oil, mineral oil, sesame oil, castor oils, polysorbates, sorbitan esters, ether sulfates, sulfates, betaines, glucosides, maltosides, fatty alcohols, nonoxinols, poloxamers, polyoxyethylenes, polyethylene glycols, dextrose, glycerol and the like. "Remington's Pharmaceutical Sciences" by E.W. Martin describes diluents, adjuvants or excipients as suitable carriers.

The following examples are merely illustrative of certain embodiments of the invention and cannot be considered as restricting it in any way.

### Abreviations

ATP: Adenosine triphosphate
EDTA: Ethylenediaminotetraacetic acid
DIPEA: *N*,*N*-Diisopropylethylamine
DCM: Dichloromethane
DMEM: Dulbecco's modified Eagle's medium
DMF: Dimethylformamide
DMSO: Dimethyl sulfoxide
DTT: Dithiothreitol ((2S,3S)-1,4-dimercaptobutane-2,3-diol)
EDTA: Ethylenediaminetetraacetic acid
EGTA: ethylene glycol-bis(2-aminoethylether)-N,N,N',N'-tetraacetic acid
FBS: Fetal bovine serum
FCS: Fetal calf serum
Fmoc: Fluorenylmethyloxycarbonyl
HPLC: High-performance liquid chromatography
HBTU: O-(Benzotriazol-1-yl)-*N*,*N*,*N*,*N*'-tetramethyluronium hexafluorophosphate
λem: emission wavelength
λex: excitation wavelength
MTT: dimethyl thiazolyl diphenyl tetrazolium salt
O.D.: Optical density
PBS: Phosphate buffered saline
SDS: Sodium dodecyl sulfate
TFA: Trifluoroacetic acid
TIS: Triisopropylsilane
TMB: tetramethylbenzidine (3,3',5,5'-tetramethylbiphenyl-4,4'-diamine)
Tris: Tris(hydroxymethyl)aminomethane
UV: Ultraviolet light

### EXAMPLES

### Example 1. Target peptide synthesis

The peptides of the invention have been prepared using the protocol described below.

In this Example AA refers to the amino acids of the peptide. The amino acids are those corresponding to the peptide of formula (I). Chemical peptide synthesis starts at the C-terminal end of the peptide and ends at the N-terminus. Therefore, the first AA is Pro for the peptide of formula (I).

Fmoc-AA-OH (3 eq) was directly incorporated on the-resin (0.5 mmol/g) with HBTU (3 eq), DIPEA (6 eq) in DMF for 1h. Washings were performed with DMF (5 x 30 s) and DCM (5 x 30 s). Kaiser test was used to verify that the coupling was successful. For the deprotection of the Fmoc group, the resin was solvated with DMF (5 x 30 s), treated with a solution of piperidine/DMF 20% (3 x 5 min) and finally washed with DMF (5 x 30 s) and DCM (5 x 30 s). Then, the resin was solvated with DMF (5 x 30 s). The same procedure was successively repeated n times with the following amino acids Fmoc-AA-OH. Finally, the cleavage of the peptide from the resin was carried out by treating the resin with TFA:TIS: H₂O (95:2.5:2.5) for 1 h, yielding the peptide of sequence HPLC: C18 column; UV 220 nm; flux 1 mL/min; gradient acetonitrile-water in 8 min.

The following peptides of sequence of formula (I) have been prepared using the protocol described:
Peptide 1: Palm-Lys-Phe-Asn-Leu-Pro-Leu-Gly-Asn-Tyr-Lys-Lys-Pro-NH₂ (Palm-SEQ ID NO: 1-NH₂)
Peptide 2: Palm-Lys-Ala-Asn-Leu-Pro-Leu-Gly-Asn-Tyr-Lys-Lys-Pro- NH₂ (Palm-SEQ ID NO: 2-NH₂)
Peptide 3: Palm-Lys-Phe-Ala-Leu-Pro-Leu-Gly-Asn-Tyr-Lys-Lys-Pro- NH₂ (Palm-SEQ ID NO: 3-NH₂)
Peptide 4: Palm-Lys-Phe-Asn-Ala-Pro-Leu-Gly-Asn-Tyr-Lys-Lys-Pro- NH₂ (Palm-SEQ ID NO: 4-NH₂)
Peptide 5: Palm-Lys-Phe-Asn-Leu-Ala-Leu-Gly-Asn-Tyr-Lys-Lys-Pro- NH₂ (Palm-SEQ ID NO: 5-NH₂)
Peptide 6: Palm-Lys-Phe-Asn-Leu-Pro-Ala-Gly-Asn-Tyr-Lys-Lys-Pro- NH₂ (Palm-SEQ ID NO: 6-NH₂)
Peptide 7: Palm-Lys-Phe-Asn-Leu-Pro-Leu-Ala-Asn-Tyr-Lys-Lys-Pro- NH₂ (Palm-SEQ ID NO: 7-NH₂)
Peptide 8: Palm-Lys-Phe-Asn-Leu-Pro-Leu-Gly-Ala-Tyr-Lys-Lys-Pro- NH₂ (Palm-SEQ ID NO: 8-NH₂)
Peptide 9: Palm-Lys-Phe-Asn-Leu-Pro-Leu-Gly-Asn-Ala-Lys-Lys-Pro- NH₂ (Palm-SEQ ID NO: 9-NH₂)
Peptide 10: Palm-Lys-Phe-Asn-Leu-Pro-Leu-Gly-Asn-Tyr-Ala-Lys-Pro- NH₂ (Palm-SEQ ID NO: 10-NH₂)
Peptide 11: Palm-Lys-Phe-Asn-Leu-Pro-Leu-Gly-Asn-Tyr-Lys-Ala-Pro- NH₂ (Palm-SEQ ID NO: 11-NH₂)
Peptide 12: Palm-Lys-Phe-Asn-Leu-Pro-Leu-Gly-Asn-Tyr-Lys-Lys-Ala- NH₂ (Palm-SEQ ID NO: 12-NH₂)

### Example 2: Preparation of the microcapsules

1 g of poly (D,L lactide-co-glycolide) (PLGA) (Resomer RG 502 H, lactide/glycolide molar ratio 48:52 to 52:48) in 10 mL of acetone containing hydrolyzed soy protein (25 mL) (Dynachondrine™) was added dropwise to 100 mL of aqueous 1% (w/v) polyvinyl alcohol (PVA), and the mixture was emulsified for 3 min using a sonicator. Following overnight evaporation of the solvent at 4 °C, the microcapsules were collected by ultracentrifugation at 60000 x g for 30 min, washed three times with distilled water, and then lyophilized for 3 to 4 days. The microcapsules obtained at this step are designated as non-targeted microcapsules in the examples.

Peptides 1 to 12 as prepared in example 1 were coupled by the N-terminus of the peptide (Lys) to the above obtained non-targerted microcapsules via amide bound to the carboxyl groups on the surface of the microcapsules present on the surface of the microcapsule (belonging to the inner polymer). Thus, carboxyl groups on the surface of the microcapsules were activated by re-suspending the microcapsules in isotonic 0.1 M 2-(N-morpholino)ethanesulphonic acid (MES) saline buffer pH 5.5, and then reacting them with 1-ethyl-3-(3-dimethyl aminopropyl) carbodiimide (EDAC) (10 equiv.) and N-hydroxysuccinimide (NHS) (10 equiv.) for 1 h. The microcapsules were then centrifuged (15000 rpm, 45 min) to remove excess EDAC/NHS and the water-soluble isourea byproduct. Activated microcapsules (1 g) were re-suspended in phosphate-buffered saline (PBS, 40 mL) and reacted with the N-terminal amine group of the peptide of sequence of formula (I) (0.150 g) at room temperature. The coated microcapsules were centrifuged (15000 rpm, 30 min) and washed with PBS (3 x 10 mL) buffer to remove any unbound peptide. The presence of surface-bound peptide was confirmed by Kaiser ninhydrin tests. Unreacted peptide was separated from the microcapsules after the corresponding reaction step by analysis of the centrifugation supernatant (step described above) using 2,4,6-trinitrobenzenesulphonic acid (TNBS) as a colorimetric assay: 700 microliters of supernatant were added to 700 microliters of 0.1 M sodium borate buffer (pH 9.2). 350 microliter of TNBS aqueous solution (1.65 mg/mL) was added and the solution was rapidly mixed. After incubation at 40 °C for 45 min., the reaction was stopped by adding 0,3 ml of 0.1 M NaH₂PO₄ containing 1.5 mM Na₂SO₃ and absorption at 420 nm was determined on a UV/VIS spectrometer. The amounts of peptide on the surface of the microcapsules were calculated by substracting the free amount from the total amount added into the reaction system, being in that case 0.1 %.

The diameter of the microcapsules was determined by Scanning Electron Microscopy (SEM) and nanosizer showing a size distribution between 180 and 400 nm.

In the examples the microcapsules obtained in accordance with the above protocol are designated with the number of the peptide used for their manufacture. That is, a microcapsule obtained using the above protocol using peptide 3 (Palm-Lys-Phe-Ala-Leu-Pro-Leu-Gly-Asn-Tyr-Lys-Lys-Pro-NH₂ (R²-SEQ ID NO: 3-R¹)) is designated as microcapsule 3.

### Example 3: Determination of the affinity for FGFR Kinases of the peptides of the invention

Evaluation of the effects of the dodecapeptides of the invention on the activity of the human FGFR1 kinase were quantified by measuring the phosphorylation of the substrate Ulight-CAGAGAIETDKEYYTVKD (JAK1) using a human recombinant enzyme expressed in insect cells and the LANCE® detection method using the protocol described below:
The peptide to be tested, the reference compound or water (control) are mixed with the enzyme (0.252 ng) in a buffer containing 40 mM Hepes/Tris (pH 7.4), 0.8 mM EGTA/Tris, 8 mM MgCl₂, 1.6 mM DTT and 0.008% Tween 20 (For control basal measurements, the enzyme is omitted from the reaction mixture). Thereafter, the reaction is initiated by adding 100 nM of the substrate Ulight-CAGAGAIETDKEYYTVKD (SEQ ID NO: 13) (JAK-1 peptide) and 100 µM ATP, and the mixture is incubated for 60 min at room temperature. Following incubation, the reaction is stopped by adding 13 mM EDTA. After 5 min, the anti-phospho-PT66 antibody labeled with europium chelate (EU-W1024-labeled Anti-Phosphotyrosine Antibody PT66, from Perkin Elmer) is added. After 60 more min, the fluorescence transfer is measured at λex=337 nm, λem=620 nm and λem=665 nm using a microplate reader (Envision, Perkin Elmer).

The enzyme activity is determined by dividing the signal measured at 665 nm by that measured at 620 nm (ratio). The results are expressed as a percent inhibition of the control enzyme activity.

The standard inhibitory reference compound is staurosporine, which is tested in each experiment at several concentrations to obtain an inhibition curve from which its IC₅₀ value is calculated.

**Table 1**

| **Peptide** | **FGFR1 Kinase % Inhibition** | **FGFR2 Kinase % Inhibition** | **FGFR3 Kinase % Inhibition** | **FGFR4 Kinase % Inhibition** |
|---|---|---|---|---|
| 1 | 0 | 95,4 | 75,8 | 38,7 |
| 2 | 0 | 96,3 | 38,8 | 96,4 |
| 3 | 85 | 98,7 | 91,9 | 65,9 |
| 4 | 0 | 87,5 | 60,8 | 0 |
| 5 | 0 | 91,1 | 49,6 | 0 |
| 6 | 92,7 | 98,8 | 78,5 | 69,8 |
| 7 | 0 | 98,4 | 72,9 | 67,9 |
| 8 | 0 | 98,8 | 87,9 | 89,5 |
| 9 | 15,7 | 99,4 | 91,6 | 92,5 |
| 10 | 0 | 93,4 | 0 | 0 |
| 11 | 0 | 89,9 | 19,3 | 0 |
| 12 | 0 | 90,1 | 46,4 | 42,3 |

Results showing an inhibition (or stimulation for assays run in basal conditions) higher than 20% are considered to represent significant effects of the peptides of the invention.

### Example 4. Cytotoxicity assays

Human dermal fibroblasts and human keratinocytes were used. The isolated cells were cultured in DMEM culture medium supplemented with 10% FCS and were incubated at 37 °C for maintenance treatment. Untreated cells were used as negative control. The positive control was 0.1% SDS. The microcapsules as obtained in Example 2 were tested in the DMEM culture medium at 0.03%. These weight percentages refer to the weight of the microcapsule in relation to the total weight.

Cells were seeded at 20000 cells per well in a 96-well plate. 24 hours after seeding, cells were incubated under the conditions described above. After 48h of incubation at 37 °C, the culture medium was removed and the MTT reagent was added. Cells were incubated 2 hours at 37 °C. DMSO was then added and the absorbance of the formed formazan salt was measured at λ 570 nm. Lower absorbance values correspond to cells with lower metabolic activity, which correlates with an increased damage and, therefore, with an increased cytotoxic effect. Thus, the amount of living cells is proportional to the amount of formazan produced. Table 2 shows the percentage of cell viability, as well as the standard deviation (SD), in the different cell lines after the different treatments.

**Table 2**

| **Tested product microcapsules targeted with the peptides** | **Fibroblasts** | | **Keratinocytes** | |
|---|---|---|---|---|
| | **% cell viability** | **SD** | **% cell viability** | **SD** |
| Control | 100 | 4 | 100 | 3 |
| SDS | 25.0 | 2 | 12 | 1 |
| Microcapsule 1 (0.003%) | 99 | 3 | 80 | 3 |
| Microcapsule 2 (0.03%) | 100 | 1 | 102 | 3 |
| Microcapsule 3 (0.03%) | 100 | 2 | 100 | 2 |
| Microcapsule 4 (0.03%) | 100 | 3 | 100 | 2 |
| Microcapsule 5 (0.03%) | 90 | 4 | 100 | 3 |
| Microcapsule 6 (0.03%) | 100 | 2 | 100 | 1 |
| Microcapsule 7 (0.03%) | 85 | 2 | 80 | 4 |
| Microcapsule 8 (0.03%) | 100 | 1 | 100 | 2 |
| Microcapsule 9 (0.03%) | 100 | 2 | 100 | 3 |
| Microcapsule 10 (0.03%) | 90 | 1 | 100 | 2 |
| Microcapsule 11 (0.03%) | 90 | 2 | 100 | 1 |
| Microcapsule 12 (0.03%) | 100 | 1 | 80 | 2 |

As can be seen, the microcapsules of Example 2 showed no changes in the cell viability at the different tested concentrations. Thus, is can be concluded that the microcapsules of Example 2 are not cytotoxic in any of the cell lines employed.

### Example 5. Cell type affinity of microcapsules of the invention

Different types of cells were incubated with microcapsules 3 (50 µg/mL) or with non-targeted microcapsules (50 µg/mL) at 37°C for 3 h. Cells were washed and stained with receptor-specific antibody or isotype control antibody to specifically detect distinct types of cells. Subsequently, cells were analyzed by flow cytometry on a FacsCalibur. The cell associated fluorescence was calculated by dividing the mean cell fluorescence intensity (mfi) for a given sample by the fibroblasts incubated with the microcapsules of the invention.

**Table 3**

| **Cell type** | **Microcapsules 3 (%)** | **Non-targeted microcapsules(%)** |
|---|---|---|
| Fibroblast | 96 ± 2 | 10 ± 2 |
| Melanocyte | 14 ± 8 | 14 ± 2 |
| Lymphocyte | 2 ± 1 | 12 ± 1 |
| Monocyte | 6 ± 1 | 8 ± 1 |
| Dendritic cell | 9 ± 1 | 14 ± 2 |
| Keratinocyte | 14 ± 2 | 10 ± 1 |

The results summarized in table 3 clearly show that the microcapsules are more selective to fibroblast that the non-targetted microcapsules.

### Example 6. Collagen and elastin synthesis in human dermal fibroblasts incubated with different boosters

### Protocol for the determination of collagen

Human dermal Fibroblasts (HDF) were harvested in 96-well plates (3000 cells by well), and they were put to grow in DMEM containing 5% of fetal bovine serum (FBS) (Invitrogen, Maryland, USA) during 24 hours. Afterwards, the medium was replaced by a fresh one containing 0.1% FBS and the relevant treatment to determine collagen production. After incubation with 50 µg/mL of non-targetted microcapsules or with 50 µg/mL of the microcapsules to be tested at 1, 2, 3, 4, 5 and 6 days, equal quantities of supernatant were added to a plate covered with human antibody of collagen type I. The plate was incubated at room temperature during 2 hours to allow the reaction between the antibody and the produced collagen. Subsequently, the plate was washed three times with PBS containing 0.5% Tween 20 to eliminate the rest of the sample that did not come together. Then, human antibody of Collagen type I was added, marked with biotin during 1 hour. After washing the plate with PBS containing 0.5% Tween 20 to eliminate unspecific interactions, it was added Streptavidin-Horseradish Peroxidase (HRP) (St Louis, Sigma). The amount of collagen type I in each well was determined using tetramethilbenzidine (St Louis, Sigma) with HRP substrate. The reaction between HRP and TMB was stopped by adding 1 N HCI, and O.D. was measured at 450 nm.

**Table 4**

| Type I Collagen (ng/ml) | | | |
|---|---|---|---|
| Time (days) | Non targeted capsules | Microcapsules 3 | % increase |
| 1 | 60 | 130 | 27 |
| 2 | 55 | 132 | 140 |
| 3 | 48 | 155 | 223 |
| 4 | 50 | 167 | 234 |
| 5 | 65 | 185 | 184 |
| 6 | 80 | 197 | 146 |

### Protocol for the determination of elastin

Human dermal Fibroblasts (HDF) were harvested in 96-well plates (3000 cells by well), and they were put to grow in DMEM containing 5% of fetal bovine serum (FBS) (Invitrogen, Maryland, USA) during 24 hours. Afterwards, the medium was replaced by a fresh one containing 0.1% FBS and 50 µg/mL of the microcapsules 1 to 12 to determine elastin production. After incubation with 50 µg/mL of non-targetted microcapsules or with 50 µg/mL of the microcapsules to be tested at 1, 2, 3, 4, 5 and 6 days, equal quantities of supernatant were added to a plate covered with human antibody of elastin. The plate was incubated at room temperature during 2 hours to allow the reaction between the antibody and the produced elastin. Subsequently, the plate was washed three times with PBS containing 0.5% Tween 20 to eliminate the rest of the sample that did not come together. Then, human antibody of elastin was added, marked with biotin during 1 hour. After washing the plate with PBS containing 0.5% Tween 20 to eliminate unspecific interactions, it was added Streptavidin-Horseradish Peroxidase (HRP) (St Louis, Sigma). The amount of Elastin in each well was determined using tetramethilbenzidine (St Louis, Sigma) with HRP substrate. The reaction between HRP and TMB was stopped by adding 1 N HCI, and O.D. was measured at 450 nm.

**Table 5**

| Elastin (ng/ml) | | | |
|---|---|---|---|
| Time (days) | Non targetted capsules | Microcapsules 3 | % increase |
| 1 | 5 | 14 | 180 |
| 2 | 7 | 17 | 143 |
| 3 | 4 | 23 | 475 |
| 4 | 5 | 26 | 420 |
| 5 | 4 | 34 | 750 |
| 6 | 7 | 45 | 543 |

The results summarized in tables 4 and 5 clearly show that the use of the microcapsules of the invention increases the production of collagen Type 1 and elastin in human dermal fibroblasts.

## Claims

1. A peptide of formula (I):
R²-Lys¹-Phe²-Asn³-Leu⁴-Pro⁵-Leu⁶-Gly-Asn⁸-Tyr⁹-Lys¹⁰-Lys¹¹-Pro¹²-R¹ (I) (R²-SEQ ID NO: 1-R¹)
or analogues thereof in which one of the aminoacid rests 2 to 12 is replaced with an alanine rest
wherein
R¹ is selected from the group consisting of -OH, -OR³, -SR³, -NR³R⁴, -OR⁵ and -NHR⁵;
R² is selected from the group consisting of H-, R³-C(O)- and R³-OC(O)-,
R³ and R⁴ are independently selected from H, C₁-C₂₄ alkyl, C₂-C₂₄ alkenyl and C₆-C₁₀ aryl;
R⁵ is selected from the group consisting
of -(CH₂)₃-PEG-CH₂-NH₂, -PEG-R⁶, - (CH₂)ₙ-O-PEG-R⁶, -CO-O-PEG-R⁶, -CO- (CH₂CH₂ )-CO-O-PEG-R⁶, -CO-(CH₂)ₘ-O-PEG-R⁶, -CO-(CH₂)ₙ-CH(R⁷) -O-PEG-R⁶, -CO-(CH₂)ₘ -CH(R⁷)-O-CO-CH(R⁷)-(CH₂)ₘ-O-PEG-R⁶ and -(1,3,5-triazine)-(O-PEG-R⁶)₂,
PEG is a polyether group selected from the group consisting of
- (CH₂-CH₂-O)ₙ-,
- (CH₂-CH(CH₃)-O)ₒ-,
- (CH(CH₃)-CH₂-O)ₒ-,
- (CH₂-CH₂-O)ₙ-(CH₂-CH(CH₃)-O)ₒ-(CH₂-CH₂-O)ₚ-,
- (CH₂-CH₂-O)ₙ-(CH(CH₃)-CH₂-O)ₒ-(CH₂-CH₂-O)ₚ-,
- (CH₂-CH₂-O)ₙ-(CH(CH₃)-CH₂-O)ₒ-,
- (CH₂-CH₂-O)ₙ-(CH₂-CH(CH₃)-O)ₒ-,
- (CH₂-CH(CH₃)-O)ₒ-(CH₂-CH₂-O)ₙ-,
- (CH(CH₃)-CH₂-O)ₒ-(CH₂-CH₂-O)ₙ-,
- (CH₂-CH(CH₃)-O)ₒ-(CH₂-CH₂-O)ₙ-(CH₂-CH(CH₃)-O)_{q}-,
- (CH₂-CH(CH₃)-O)ₒ-(CH₂-CH₂-O)ₙ-(CH(CH₃)-CH₂-O)_{q}-,
- (CH(CH₃)-CH₂O)ₒ-(CH₂-CH₂-O)ₙ-(CH₂-CH(CH₃)-O)_{q}- and
- (CH(CH₃)-CH₂O)ₒ-(CH₂-CH₂-O)ₙ-(CH(CH₃)-CH₂-O)ₒ-,
R⁶ is a hydrogen atom or a C₁₋₄ alkyl group; and
R⁷ is a C₁₋₄ alkyl group
m is an integer from 1 to 4
n and p are integers between 1 and 10
m and o are integers between 1 and 10
and cosmetically acceptable salts and solvates thereof.

2. A microcapsule comprising a peptide as defined in claim 1, coupled to the outer surface thereof.

3. A microcapsule according to claim 2 additionally comprising an antiaging compound selected from the group consisting of peptides; olive leaf extract, oleanolic acid, oleanol, green tea extract, acerola extract, creatine, extracts from maize kernels, extracts of black elderberry, extract from cocoa beans, extracts of peppermint, dihydroquercetin (taxifolin), Ginger root extract, Galanga, dill extract, Ginger Root, or Zingiber Officinale Root Extract (COX-2 inhibitor), Galanga, or Alpinia Officinarum Extract, Turmeric, or Curcuma Longa Root Extract, Mango Ginger, or Curcuma amada, Capsicum, or Capsicum Annuum Extract, Clove Family, or Syzygium Aromaticum Extract, Evodia, or Evodia Rutaecarpa Fruit Extract, Boswellia, or Boswellia Serrata Extract, SAMe, or S-Adenosylmethionine, Eucomis, or Eucomis L"Herit, Celastrus, or Celastrus orbiculatus, Tithonia, or Tithonia diversifolia, Kochia, or Kochia Scoparia Extract, Scoparia, or Scoparia dulcis Extract, Qiang Huo, or Notopterygium incisum, Cinnamon or Cinnamonum cassia extract, Mexican Bamboo or Polygonum cuspidatum extract, Ogon, Baikal Scullcap, or Scutellaria baicalensis extract, Coptis, Xianglian, or Coptis chinenesis extracts, Psoralea, Rumex extract, Baccharis extract, Feverfew extract, Vitis, Stephania extract, Corydalis or Corydalis Turtschaninovii Root Extract, Horse Chestnut Extract (Aesculus hippocastanum extract)), Esculin, Escin, Yohimbine, Capsicum Oleoresin extract, Capsaicin, Niacin, Niacin Esters, Methyl Nicotinate, Benzyl Nicotinate, Ruscogenins (Butchers Broom extract; Ruscus aculeatus extract), Diosgenin (Trigonella foenumgraecum, Fenugreek), Emblica extract (Phyllanthus emblica extract), Asiaticoside (Centella asiatica extract), Boswellia Extract (Boswellia serrata), Ginger Root Extract (Zingiber Officianalis), Piperine, Vitamin K, Melilot (Melilotus officinalis extract), Glycyrrhetinic acid, Ursolic acid, Sericoside (Terminalia sericea extract), Darutoside (Siegesbeckia orientalis extract), Amni visnaga extract, extract of Red Vine (Vitis-Vinifera) leaves, apigenin, phytosan, and luteolin; vitamins selected from the group consisting of vitamin A, vitamin B, vitamin C, vitamin E, vitamin H and vitamin K, green tea extract, resveratrol, elagic acid; coenzymes Q10, superoxidodismutase, catalase, glutathione peroxidase, etc; flavonoids selected from the group consisting of acacetin, apigenin, baicalein, chrysin, diosmetin, diosmin, galangin, hydroxyflavones, isorhamnetin, kaempferol, luteolin, flavanes, isoflavones, queratin, ubiquinone,ubiquinol, silymarin, ectoine, hyaluronic acid; retinoic acid, retinaldehyde, retinol, retinyl, retinoate, retinyl esters, melatonin; resveratrol, Oligomeric proanthocyanidins (OPC), liquorice extract, alpha-bisabolol, azulene, glycyrrhetic acid, aloe vera extract and rosmarinum oficinalis extract; gingerol, shogaol, zingerone, and capsaicin, Horse Chestnut Extract (Aesculus hippocastanum extract)), Esculin, Escin, Yohimbine, Capsicum Oleoresin, Capsaicin, Niacin, Niacin Esters, Methyl Nicotinate, Benzyl Nicotinate,; oroxylum and catechin.

4. A microcapsule according to anyone of claims 2 to 3, **characterized in that** it has at least one shell comprising a polymeric material.

5. A microcapsule according to anyone of claims 2 to 4 **characterized in that** it is a bilayered polymeric microcapsule and wherein the polymers forming the microcapsule's shell are selected from the group consisting of poly (D,L-lactide-co-glycolide), poly lactic acids, poly (propylene fumarate-co-ethylene glycol) [P(PF-co-EG)] block copolymer, poly-anhydride poly (fumaric-co-sebacic) anhydride, poly (ethylene oxide)-poly (lactide/glycolide), poly(amino acid), polyvinyl alcohol, alginate, dextran, chitosan, hydroxyapatite, collagen, fibrin, hyaluronic acid, carbomers, poly(amino acid), poly(ethylene glycol).

6. The microcapsule according to claim 5, **characterized in that** the bilayered polymeric microcapsule comprises poly (D,L-lactide-co-glycolide) as inner layer polymer and polyvinyl alcohol as outer layer polymer.

7. The microcapsule according to anyone of claims 2 to 6, **characterized in that** the compound of formula (I) is covalently coupled to the outer layer polymer of the microcapsule.

8. The microcapsule according to anyone of claims 1 to 7, **characterized in that** it has a size ranging from 10 to 10000 nm.

9. A process for obtention of the microcapsules as defined in claims 2 to 8 comprising the steps of
a) forming the microcapsule encapsulating the antiaging compound and
b) coupling a compound of formula (I):
R²-Lys¹-Phe²-Asn³-Leu⁴-Pro⁵-Leu⁶-Gly-Asn⁸-Tyr⁹-Lys¹⁰-Lys¹¹-Pro¹²-R¹ (I) (R²-SEQ ID NO: 1-R¹)
or analogues thereof in which one of the aminoacid rests 2 to 12 is replaced with an alanine rest
wherein
R¹ is selected from the group consisting of -OH, -OR³, -SR³, -NR³R⁴, -OR⁵ and -NHR⁵,
R² is selected from the group consisting of H-, R³-C(O)- and R³-OC(O)-,
R³ and R⁴ are independently selected from H, C₁-C₂₄ alkyl, C₂-C₂₄ alkenyl and C₆-C₁₀ aryl;
R⁵ is selected from the group consisting
of -(CH₂)₃-PEG-CH₂-NH₂, -PEG-R⁶, - (CH₂)ₙ-O-PEG-R⁶, -CO-O-PEG-R⁶, -CO- (CH₂CH₂ )-CO-O-PEG-R⁶, -CO-(CH₂)ₘ-O-PEG-R⁶, -CO-(CH₂)ₙ-CH(R⁷) -O-PEG-R⁶, -CO-(CH₂)ₘ -CH(R⁷)-O-CO-CH(R⁷)-(CH₂)ₘ-O-PEG-R⁶ and -(1,3,5-triazine)-(O-PEG-R⁶)₂,
PEG is a polyether group selected from the group consisting of
- (CH₂-CH₂-O)ₙ-,
- (CH₂-CH(CH₃)-O)ₒ-,
- (CH(CH₃)-CH₂-O)ₒ-,
- (CH₂-CH₂-O)ₙ-(CH₂-CH(CH₃)-O)ₒ-(CH₂-CH₂-O)ₚ-,
- (CH₂-CH₂-O)ₙ-(CH(CH₃)-CH₂-O)ₒ-(CH₂-CH₂-O)ₚ-,
- (CH₂-CH₂-O)ₙ-(CH(CH₃)-CH₂-O)ₒ-,
- (CH₂-CH₂-O)ₙ-(CH₂-CH(CH₃)-O)ₒ-,
- (CH₂-CH(CH₃)-O)ₒ-(CH₂-CH₂-O)ₙ-,
- (CH(CH₃)-CH₂-O)ₒ-(CH₂-CH₂-O)ₙ-,
- (CH₂-CH(CH₃)-O)ₒ-(CH₂-CH₂-O)ₙ-(CH₂-CH(CH₃)-O)_{q}-,
- (CH₂-CH(CH₃)-O)ₒ-(CH₂-CH₂-O)ₙ-(CH(CH₃)-CH₂-O)_{q}-,
- (CH(CH₃)-CH₂O)ₒ-(CH₂-CH₂-O)ₙ-(CH₂-CH(CH₃)-O)_{q}- and
- (CH(CH₃)-CH₂O)ₒ-(CH₂-CH₂-O)ₙ-(CH(CH₃)-CH₂-O)ₒ-,
R⁶ is a hydrogen atom or a C₁₋₄ alkyl group; and
R⁷ is a C₁₋₄ alkyl group
m is an integer from 1 to 4
n and p are integers between 1 and 10
m and o are integers between 1 and 10
and cosmetically acceptable salts and solvates thereof, to the outer surface of the microcapsule after forming the microcapsule.

10. The process according to claim 9, wherein the microcapsule is a polymeric bilayered microcapsule having an inner layer polymer and an outer layer polymer and the step a) of forming the microcapsule comprises the steps of
a1) mixing the inner layer polymer with the antiaging compound in a suitable solvent,
a2) emulsifying the mixture obtained in step a) with the outer layer polymer in a suitable solvent, and optionally
a3) isolating the microcapsules.

11. A cosmetic composition comprising the microcapsule as defined in anyone of claims 2 to 10.

12. Cosmetic use of a peptide as defined in claim 1 for promoting the synthesis of collagen type I and or the synthesis of elastin.

13. Use of the microcapsules as defined in anyone of claims 2 to 10 for the cosmetic prevention and/or cosmetic treatment of sking aging.

14. Use of the microcapsules as defined in any of claims 2 to 10 to improve skin aspect by achieving one or more of the following effects: increasing luminosity of skin, increasing skin elasticity, diminishing skin roughness, reducing skin wrinkles, increasing apparent skin thinning, reducing skin pigmentation defaults.

## Patentansprüche

1. Peptid der Formel (I):
R²-Lys¹-Phe²-Asn³-Leu⁴-Pro⁵-Leu⁶-Gly⁷-Asn⁸-Tyr⁹-Lys¹⁰-Lys¹¹-Pro¹²-R¹ (I) (R²-SEQ ID NO: 1-R¹)
oder Analoge davon, in denen einer der Aminosäurereste 2 bis 12 durch einen Alaninrest ersetzt ist,
wobei
R¹ ausgewählt ist aus der Gruppe, bestehend aus -OH, -OR³, -SR³, -NR³R⁴, -OR⁵ und -NHR⁵;
R² ausgewählt ist aus der Gruppe, bestehend aus H-, R³-C(O)- und R³-OC(O)-,
R³ und R⁴ unabhängig ausgewählt sind aus H, C₁-C₂₄-Alkyl, C₂-C₂₄-Alkenyl und C₆-C₁₀-Aryl;
R⁵ ausgewählt ist aus der Gruppe, bestehend aus -(CH₂)₃PEG-CH₂-NH₂, -PEG-R⁶, -(CH₂)ₙ-O-PEG-R⁶, -CO-O-PEG-R⁶, -CO-(CH₂CH₂)-CO-O-PEG-R⁶, -CO-(CH₂)ₘ-O-PEG-R⁶, -CO-(CH₂)ₙ-CH(R⁷)-O-PEG-R⁶, -CO-(CH₂)ₘ-CH(R⁷)-O-CO-CH(R⁷)-(CH₂) ₘ-O-PEG-R⁶ und -(1,3,5-Triazin)-(O-PEG-R⁶)₂,
PEG eine Polyethergruppe ist, ausgewählt aus der Gruppe, bestehend aus
- (CH₂-CH₂-O)ₙ-,
- (CH₂-CH(CH₃)-O)ₒ-,
- (CH(CH₃)-CH₂-O)ₒ-,
- (CH₂-CH₂-O)ₙ-(CH₂-CH(CH₃)-O)ₒ-(CH₂-CH₂-O)ₚ-,
- (CH₂-CH₂-O)ₙ-(CH(CH₃)-CH₂-O)ₒ-(CH₂-CH₂-O)ₚ-,
- (CH₂-CH₂-O)ₙ-(CH(CH₃)-CH₂-O)ₒ-,
- (CH₂-CH₂-O)ₙ-(CH₂-CH(CH₃)-O)ₒ-,
- (CH₂-CH(CH₃)-O)ₒ-(CH₂-CH₂-O)ₙ-,
- (CH(CH₃)-CH₂-O)ₒ-(CH₂-CH₂-O)ₙ-,
- (CH₂-CH(CH₃)-O)ₒ-(CH₂-CH₂-O)ₙ-(CH₂-CH(CH₃)-O)_{q}-,
- (CH₂-CH(CH₃)-O)ₒ-(CH₂-CH₂-O)ₙ-(CH(CH₃)-CH₂-O)_{q}-,
- (CH(CH₃)-CH₂O)ₒ-(CH₂-CH₂-O)ₙ-(CH₂-CH(CH₃)-O)_{q}- und
- (CH(CH₃)-CH₂O)ₒ-(CH₂-CH₂-O)ₙ-(CH(CH₃)-CH₂-O)ₒ-,
R⁶ ein Wasserstoffatom oder eine C₁₋₄-Alkylgruppe ist; und
R⁷ eine C₁₋₄-Alkylgruppe ist,
m eine ganze Zahl von 1 bis 4 ist,
n und p ganze Zahlen zwischen 1 und 10 sind,
m und o ganze Zahlen zwischen 1 und 10 sind,
und kosmetisch akzeptable Salze und Solvate davon.

2. Mikrokapsel, umfassend ein Peptid, wie in Anspruch 1 definiert, das an die äußere Oberfläche davon gekoppelt ist.

3. Mikrokapsel nach Anspruch 2, das zusätzlich eine Antialterungs-Verbindung umfasst, ausgewählt aus der Gruppe, bestehend aus Peptiden; Olivenblattextrakt, Oleanolsäure, Oleanol, Grüntee-Extrakt, Acerolaextrakt, Kreatin, Extrakte von Maiskörnern, Extrakte von schwarzem Holunder, Extrakte von Kakaobohnen, Extrakte von Pfefferminz, Dihydroquercetin (Taxifolin), Ingwerwurzel-Extrakt, Galanga, Dill-Extrakt, Ingwerwurzel, oder Zingiber Officinale-Wurzelextrakt (COX-2-Inhibitor), Galanga, oder Alpinia Officinarum-Extrakt, Curcuma, oder Curcuma Longa Wurzel-Extrakt, Mango-Ingwer, oder Curcuma Amada, Capsicum, oder Capsicum Annuum-Extrakt, Nelkenfamilie, oder Syzygium Aromaticum-Extrakt, Evodia, oder Evodia Rutaecarpa-Frucht-Extrakt, Boswellia, oder Boswellia Serrata-Extrakt, SAMe, oder S-Adenosylmethionin, Eucomis, oder Eucomis L"Herit, Celastrus, oder Celastrus orbiculatus, Tithonia, oder Tithonia diversifolia, Kochia, oder Kochia Scoparia-Extrakt, Scoparia, oder Scoparia dulcis-Extrakt, Qiang Huo, oder Notopterygium incisum, Zimt oder Cinnamonum cassia-Extrakt, mexikanischer Bambus oder Polygonum cuspidatum-Extrakt, Ogon, Baikal-Helmkraut, oder Scutellaria baicalensis-Extrakt, Coptis, Xianglian, oder Coptis chinenesis-Extrakte, Psoralea, Rumex-Extrakt, Baccharis-Extrakt, Mutterkraut-Extrakt, Vitis, Stephania-Extrakt, Corydalis oder Corydalis Turtschaninovii-Wurzel-Extrakt, Rosskastanien-Extrakt (Aesculus hippocastanum-Extrakt)), Esculin, Escin, Yohimbin, Capsicum Oleoresin-Extrakt, Capsaicin, Niacin, NiacinEster, Methylnicotinat, Benzylnicotinat, Ruscogenine (stechender Mäusedorn-Extrakt; Ruscus aculeatus-Extrakt), Diosgenin (Trigonella foenumgraecum, Bockshornklee), Emblica-Extrakt (Phyllanthus emblica-Extrakt), Asiaticosid (Centella asiatica-Extrakt), Boswellia-Extrakt (Boswellia serrata), Ingwerwurzel-Extrakt (Zingiber Officianalis), Piperin, Vitamin K, Steinklee (Melilotus officinalis-Extrakt), Glycyrrhizinsäure, Ursolsäure, Sericosid (Terminalia sericea-Extrakt), Darutosid (Siegesbeckia orientalis-Extrakt), Amni visnaga-Extrakt, Extrakt von roten Weinblättern (Vitis-Vinifera), Apigenin, Phytosan, und Luteolin; Vitamine, ausgewählt aus der Gruppe, bestehend aus Vitamin A, Vitamin B, Vitamin C, Vitamin E, Vitamin H und Vitamin K, Grüntee-Extrakt, Resveratrol, Ellagsäure; Coenzyme Q₁₀, Superoxidodismutase, Catalase, Glutathion-Peroxidas, etc.;
Flavonoide, ausgewählt aus der Gruppe, bestehend aus Acacetin, Apigenin, Baicalein, Chrysin, Diosmetin, Diosmin, Galangin, Hydroxyflavone, Isorhamnetin, Kaempferol, Luteolin, Flavane, Isoflavone, Queratin, Ubichinon, Ubichinol, Silymarin, Ectoin, Hyaluronsäure; Retinoidsäure, Retinaldehyd, Retinol, Retinyl, Retinoat, Retinylester, Melatonin; Resveratrol, Oligomere Proanthocyanidine (OPC), Lakritz-Extrakt, Alpha-Bisabolol, Azulen, Glycyrrhizinsäure, Aloe Vera-Extrakt und Rosmarinum oficinalis-Extrakt; Gingerol, Shogaol, Zingeron, und Capsaicin, Rosskastanien-Extrakt (Aesculus hippocastanum-Extrakt)), Esculin, Escin, Yohimbin, Capsicum Oleoresin, Capsaicin, Niacin, Niacinester, Methylnicotinat, Benzylnicotinat; Oroxylum und Catechin.

4. Mikrokapsel nach einem der Ansprüche 2 bis 3, **dadurch gekennzeichnet, dass** sie wenigstens eine Schale hat, umfassend ein polymeres Material.

5. Mikrokapsel nach einem der Ansprüche 2 bis 4, **dadurch gekennzeichnet, dass** sie eine zweischichtige polymere Mikrokapsel ist, und wobei die Polymere, die die Schale der Mikrokapsel bilden, ausgewählt sind aus der Gruppe, bestehend aus Poly(D,L-lactid-co-glycolid), Polymilchsäuren, Poly(propylenfumarat-co-ethylenglycol) [P(PF-co-EG)] Block-copolymer, Poly-anhydrid-poly(fumar-co-sebacin) anhydrid, Poly(ethylenoxid)-Poly(lactid/glycolid), Poly(aminosäure), Polyvinylalkohol, Alginat, Dextran, Chitosan, Hydroxylapatit, Collagen, Fibrin, Hyaluronsäure, Carbomere, Poly(aminosäure), Poly(ethylenglycol).

6. Mikrokapsel nach Anspruch 5, **dadurch gekennzeichnet, dass** die zweischichtige polymere Mikrokapsel Poly(D,L-lactid-co-glycolid) als Polymer der inneren Schicht und Polyvinylalkohol als Polymer der äußeren Schicht umfasst.

7. Mikrokapsel nach einem der Ansprüche 2 bis 6, **dadurch gekennzeichnet, dass** die Verbindung der Formel (I) kovalent an das Polymer der äußeren Schicht der Mikrokapsel gekoppelt ist.

8. Mikrokapsel nach einem der Ansprüche 1 bis 7, **dadurch gekennzeichnet, dass** sie eine Größe im Bereich von 10 bis 10000 nm hat.

9. Verfahren zum Erhalt der Mikrokapseln, wie in Ansprüchen 2 bis 8 definiert, umfassend die Schritte
a) Bilden der Mikrokapsel, die die Antialterungs-Verbindung verkapselt, und
b) Koppeln einer Verbindung der Formel (1):
R²-Lys¹-Phe²-Asn³-Leu⁴-Pro⁵-Leu⁶-Gly⁷-Asn⁸-Tyr⁹-Lys¹⁰-Lys¹¹-Pro¹²-R¹ (I) (R²-SEQ ID NO: 1-R¹)
oder Analoge davon, in denen einer der Aminosäurereste 2 bis 12 durch einen Alaninrest ersetzt ist,
wobei
R¹ ausgewählt ist aus der Gruppe, bestehend aus -OH, -OR³, -SR³, -NR³R⁴, -OR⁵ und -NHR⁵,
R² ausgewählt ist aus der Gruppe, bestehend aus H-, R³-C(O)- und R³-OC(O)-,
R³ und R⁴ unabhängig ausgewählt sind aus H, C₁-C₂₄-Alkyl, C₂-C₂₄-Alkenyl und C₆-C₁₀-Aryl;
R⁵ ausgewählt ist aus der Gruppe, bestehend aus -(CH₂)₃-PEG-CH₂-NH₂, -PEG-R⁶, -(CH₂)ₙ-O-PEG-R⁶, -CO-O-PEG-R⁶, -CO-(CH₂CH₂)-CO-O-PEG-R⁶, -CO-(CH₂)ₘ-O-PEG-R⁶, -CO-(CH₂)ₙ-CH(R⁷)-O-PEG-R⁶, -CO-(CH₂)ₘ-CH(R⁷)-O-CO-CH(R⁷)-(CH₂) ₘ-O-PEG-R⁶ und -(1,3,5-Triazin)-(O-PEG-R⁶)₂,
PEG eine Polyethergruppe ist, ausgewählt ist aus der Gruppe, bestehend aus
- (CH₂-CH₂-O)ₙ-,
- (CH₂-CH(CH₃)-O)ₒ-,
- (CH(CH₃)-CH₂-O)ₒ-,
- (CH₂-CH₂-O)ₙ-(CH₂-CH(CH₃)-O)ₒ-(CH₂-CH₂-O)ₚ-,
- (CH₂-CH₂-O)ₙ-(CH(CH₃)-CH₂-O)ₒ-(CH₂-CH₂-O)ₚ-,
- (CH₂-CH₂-O)ₙ-(CH(CH₃)-CH₂-O)ₒ-,
- (CH₂-CH₂-O)ₙ-(CH₂-CH(CH₃)-O)ₒ-,
- (CH₂-CH(CH₃)-O)ₒ-(CH₂-CH₂-O)ₙ-,
- (CH(CH₃)-CH₂-O)ₒ-(CH₂-CH₂-O)ₙ-,
- (CH₂-CH(CH₃)-O)ₒ-(CH₂-CH₂-O)ₙ-(CH₂-CH(CH₃)-O)_{q}-,
- (CH₂-CH(CH₃)-O)ₒ-(CH₂-CH₂-O)ₙ-(CH(CH₃)-CH₂-O)_{q}-,
- (CH(CH₃)-CH₂O)ₒ-(CH₂-CH₂-O)ₙ-(CH₂-CH(CH₃)-O)_{q}- und
- (CH(CH₃)-CH₂O)ₒ-(CH₂-CH₂-O)ₙ-(CH(CH₃)-CH₂-O)ₒ-,
R⁶ ein Wasserstoffatom oder eine C₁₋₄-Alkylgruppe ist; und
R⁷ eine C₁₋₄-Alkylgruppe ist,
m eine ganze Zahl von 1 bis 4 ist,
n und p ganze Zahlen zwischen 1 und 10 sind,
m und o ganze Zahlen zwischen 1 und 10 sind,
und kosmetisch akzeptable Salze und Solvate davon,
an die äußere Oberfläche der Mikrokapsel nach Bilden der Mikrokapsel.

10. Verfahren nach Anspruch 9, wobei die Mikrokapsel eine polymere zweischichtige Mikrokapsel mit einem Polymer der inneren Schicht und einem Polymer der äußeren Schicht ist, und wobei der Schritt a) des Bildens der Mikrokapsel die Schritte umfasst
a1) Mischen des Polymers der inneren Schicht mit der Antialterungs-Verbindung in einem geeigneten Lösungsmittel,
a2) Emulgieren der in Schritt a) erhaltenen Mischung mit dem Polymer der äußeren Schicht in einem geeigneten Lösungsmittel, und optional
a3) Isolieren der Mikrokapseln.

11. Kosmetische Zusammensetzung, umfassend die Mikrokapsel, wie in einem der Ansprüche 2 bis 10 definiert.

12. Kosmetische Verwendung eines Peptids, wie in Anspruch 1 definiert, zum Fördern der Synthese von Typ I Collagen und oder der Synthese von Elastin.

13. Verwendung der Mikrokapseln, wie in einem der Ansprüche 2 bis 10 definiert, zur kosmetischen Verhinderung und/oder kosmetischen Behandlung der Hautalterung.

14. Verwendung der Mikrokapseln, wie in einem der Ansprüche 2 bis 10 definiert, zum Verbessern des Aussehens der Haut durch Erzielen von einem oder mehreren der folgenden Effekte: Erhöhen der Leuchtkraft der Haut, Erhöhen der Hautelastizität, Verringern der Hautrauheit, Verringern von Hautfalten, Erhöhen der sichtbaren Hautverdünnung, Reduzieren von Hautpigmentierungsfehlern.

## Revendications

1. Peptide de formule (I) :
R²-Lys¹-Phe²-Asn³-Leu⁴-Pro⁵-Leu⁶-Gly⁷-Asn⁸-Tyr⁹-Lys¹⁰-Lys¹¹-Pro¹²-R¹ (I) (R²-SEQ ID NO : 1-R¹)
ou des analogues de celui-ci dans lesquels l'un des résidus acides aminés 2 à 12 est remplacé par un résidu alanine
où
R¹ est choisi dans le groupe constitué par -OH, -OR³, -SR³, -NR³R⁴, -OR⁵ et -NHR⁵;
R² est choisi dans le groupe constitué par H-, R³-C(O)- et R³-OC(O)-,
R³ et R⁴ sont indépendamment choisis parmi H, alkyle en C₁-C₂₄, alcényle en C₂-C₂₄ et aryle en C₆-C₁₀ ;
R⁵ est choisi dans le groupe constitué par -(CH₂)₃-PEG-CH₂-NH₂, -PEG-R⁶, -(CH₂)ₙ-O-PEG-R⁶, -CO-O-PEG-R⁶, -CO-(CH₂CH₂)-CO-O-PEG-R⁶, -CO-(CH₂)ₘ-O-PEG-R⁶, -CO-(CH₂)ₙ-CH(R⁷)-O-PEG-R⁶, -CO-(CH₂)ₘ₋CH(R⁷)-O-CO-CH(R⁷)-(CH₂)ₘ₋O-PEG-R⁶ et - (1,3,5-triazine)-(O-PEG-R⁶)₂,
PEG est un groupe de polyéther choisi dans le groupe constitué par
- (CH₂-CH₂-O)ₙ-,
- (CH₂-CH(CH₃)-O)ₒ-,
- (CH(CH₃)-CH₂-O)ₒ-,
- (CH₂-CH₂-O)ₙ-(CH₂-CH(CH₃)-O)ₒ-(CH₂-CH₂-O)ₚ-,
- (CH₂-CH₂-O)ₙ-(CH(CH₃)-CH₂-O)ₒ-(CH₂-CH₂-O)ₚ-,
- (CH₂-CH₂-O)ₙ-(CH(CH₃)CH₂-O)ₒ-,
- (CH₂-CH₂-O)ₙ-(CH₂-CH(CH₃)-O)ₒ-,
- (CH₂-CH(CH₃)-O)ₒ-(CH₂-CH₂-O)ₙ-,
- (CH(CH₃)-CH₂-O)ₒ-(CH₂-CH₂-O)ₙ-,
- (CH₂-CH(CH₃)-O)ₒ-(CH₂-CH₂-O)ₙ-(CH₂-CH(CH₃)-O)_{q}-,
- (CH₂-CH(CH₃)-O)ₒ-(CH₂-CH₂-O)ₙ-(CH(CH₃)-CH₂-O)_{q}-,
- (CH(CH₃)-CH₂O)ₒ-(CH₂-CH₂-O)ₙ-(CH₂-CH(CH₃)-O)_{q}- et
- (CH(CH₃)-CH₂O)ₒ-(CH₂-CH₂-O)ₙ-(CH(CH₃)-CH₂-O)ₒ-,
R⁶ est un atome d'hydrogène ou un groupe alkyle en C₁₋₄ ; et
R⁷ est un groupe alkyle en C₁₋₄
m est un nombre entier allant de 1 à 4
n et p sont des nombres entiers compris entre 1 et 10
m et o sont des nombres entiers compris entre 1 et 10
et des sels et solvates cosmétiquement acceptables de ceux-ci.

2. Microcapsule comprenant un peptide tel que défini dans la revendication 1, couplé à la surface externe de celle-ci.

3. Microcapsule selon la revendication 2, comprenant de plus un composé antivieillissement choisi dans le groupe constitué par les peptides ; l'extrait de feuille d'olivier, l'acide oléanolique, l'oléanol, l'extrait de thé vert, l'extrait d'acérola, la créatine, les extraits provenant de grains de maïs, les extraits de baie de sureau noir, l'extrait provenant de fèves de cacao, les extraits de menthe poivrée, la dihydroquercétine (taxifoline), l'extrait de racine de gingembre, le galanga, l'extrait d'aneth, l'extrait de racine de gingembre, ou de racine de Zingiber officinale (inhibiteur de COX-2), l'extrait de galanga, ou Alpinia officinarum, l'extrait de racine de curcuma, ou Curcuma longa, le gingembre mangue, ou Curcuma amada, l'extrait de capsicum, ou Capsicum annuum, l'extrait de la famille des clous de girofle, ou Syzygium aromaticum, l'extrait de fruit Evodia, ou Evodia rutaecarpa, l'extrait de Boswellia, ou Boswellia serrata, la SAMe, ou S-Adénosylméthionine, l'Eucomis, ou Eucomis L'Herit, le Celastrus, ou Celastrus orbiculatus, le Tithonia, ou Tithonia diversifolia, l'extrait de Kochia, ou Kochia scoparia, l'extrait de Scoparia, ou Scoparia dulcis, le Qiang Huo, ou Notopterygium incisum, l'extrait de cannelle ou Cinnamonum cassia, l'extrait de bambou mexicain ou Polygonum cuspidatum, l'ogon, l'extrait de scutellaire du Baïkal, ou Scutellaria baicalensis, les extraits de Coptis, Xianglian, ou Coptis chinenesis, le Psoralea, l'extrait de Rumex, l'extrait de Baccharis, l'extrait de chrysanthème matricaire, le Vitis, l'extrait de Stephania, l'extrait de racine de corydalis ou Corydalis Turtschaninovii, l'extrait de marronnier d'Inde (extrait d'Aesculus hippocastanum), l'esculine, l'escine, la yohimbine, l'extrait d'oléorésine de Capsicum, la capsaïcine, la niacine, les esters de niacine, le nicotinate de méthyle, le nicotinate de benzyle, les ruscogénines (extrait de fragon ; extrait de Ruscus aculeatus), la diosgénine (Trigonella foenumgraecum, fenugrec), l'extrait d'emblica (extrait de Phyllanthus emblica), l'asiaticoside (extrait de Centella asiatica), l'extrait de Boswellia (Boswellia serrata), l'extrait de racine de gingembre (Zingiber Officianalis), la pipérine, la vitamine K, le mélilot (extrait de Melilotus officinalis), l'acide glycyrrhétinique, l'acide ursolique, le séricoside (extrait de Terminalia sericea), le darutoside (extrait de Siegesbeckia orientalis), l'extrait d'Amni visnaga, l'extrait de feuilles de vigne rouge (Vitis-Vinifera), l'apigénine, le phytosan, et la lutéoline ; les vitamines choisies dans le groupe constitué par la vitamine A, la vitamine B, la vitamine C, la vitamine E, la vitamine H et la vitamine K, l'extrait de thé vert, le resvératrol, l'acide ellagique ; les coenzymes Q10, la superoxidodismutase, la catalase, la glutathion peroxydase, etc.; les flavonoïdes choisis dans le groupe constitué par l'acacétine, l'apigénine, la baicaléine, la chrysine, la diosmétine, la diosmine, la galangine, les hydroxyflavones, l'isorhamnétine, le kaempférol, la lutéoline, les flavanes, les isoflavones, la kératine, l'ubiquinone, l'ubiquinol, la silymarine, l'ectoïne, l'acide hyaluronique ; l'acide rétinoïque, le rétinaldehyde, le rétinol, le rétinyle, le rétinoate, les esters de rétinyle, la mélatonine ; le resvératrol, les proanthocyanidines oligomères (OPC), l'extrait de réglisse, l'alpha-bisabolol, l'azulène, l'acide glycyrrhétique, l'extrait d'aloe vera et l'extrait de Rosmarinum oficinalis ; le gingérol, le shogaol, la zingérone, et la capsaïcine, l'extrait de marronnier d'Inde (extrait d'Aesculus hippocastanum), l'esculine, l'escine, la yohimbine, l'oléorésine de Capsicum, la capsaïcine, la niacine, les esters de niacine, le nicotinate de méthyle, le nicotinate de benzyle ; l'oroxylum et la catéchine.

4. Microcapsule selon l'une quelconque des revendications 2 à 3, **caractérisée en ce qu'**il a au moins une enveloppe comprenant un matériau polymère.

5. Microcapsule selon l'une quelconque des revendications 2 à 4, **caractérisée en ce qu'**elle est une microcapsule polymère à double couche et où les polymères formant l'enveloppe de la microcapsule sont choisis dans le groupe constitué par le poly (D,L-lactide-co-glycolide), les acides poly lactiques, un copolymère à blocs de poly (fumarate de propylène-co-éthylène glycol) [P(PF-co-EG)], un poly-anhydride poly anhydride (fumarique-co-sébacique), un poly(oxyde d'éthylène)-poly (lactide/glycolide), un poly(acide aminé), l'alcool polyvinylique, l'alginate, le dextrane, le chitosane, l'hydroxyapatite, le collagène, la fibrine, l'acide hyaluronique, les carbomères, un poly(acide aminé), le poly(éthylène glycol).

6. Microcapsule selon la revendication 5, **caractérisée en ce que** la microcapsule polymère à double couche comprend du poly (D,L-lactide-co-glycolide) comme polymère de couche interne et de l'alcool polyvinylique comme polymère de couche externe.

7. Microcapsule selon l'une quelconque des revendications 2 à 6, **caractérisée en ce que** le composé de formule (I) est couplé de manière covalente au polymère de couche externe de la microcapsule.

8. Microcapsule selon l'une quelconque des revendications 1 à 7, **caractérisée en ce qu'**elle a une dimension allant de 10 à 10 000 nm.

9. Procédé d'obtention des microcapsules telles que définies dans les revendications 2 à 8, comprenant les étapes consistant à
a) former la microcapsule encapsulant le composé antivieillissement et
b) coupler un composé de formule (I) :
R²⁻Lys¹-Phe²-Asn³-Leu⁴-Pro⁵-Leu⁶-Gly⁷-Asn⁸-Tyr⁹-Lys¹⁰-Lys¹¹-Pro¹²-R¹ (I) (R²-SEQ ID NO : 1-R¹)
ou des analogues de celui-ci dans lesquels l'un des résidus acides aminés 2 à 12 est remplacé par un résidu alanine
où
R¹ est choisi dans le groupe constitué par -OH, -OR³, -SR³, -NR³R⁴, -OR⁵ et -NHR⁵;
R² est choisi dans le groupe constitué par H-, R³-C(O)- et R³-OC(O)-,
R³ et R⁴ sont indépendamment choisis parmi H, alkyle en C₁-C₂₄, alcényle en C₂-C₂₄ et aryle en C₆-C₁₀ ;
R⁵ est choisi dans le groupe constitué par -(CH₂)₃-PEG-CH₂-NH₂, -PEG-R⁶, -(CH₂)ₙ-O-PEG-R⁶, -CO-O-PEG-R⁶, -CO-(CH₂CH₂)-CO-O-PEG-R⁶, -CO-(CH₂)ₘ-O-PEG-R⁶, -CO-(CH₂)ₙ-CH(R⁷)-O-PEG-R⁶, -CO-(CH₂)ₘ-CH(R⁷)-O-CO-CH(R⁷)-(CH₂)ₘ-O-PEG-R⁶ et -(1,3,5-triazine)-(O-PEG-R⁶)₂,
PEG est un groupe de polyéther choisi dans le groupe constitué par
- (CH₂-CH₂-O)ₙ-,
- (CH₂-CH(CH₃)-O)ₒ-,
- (CH(CH₃)-CH₂-O)ₒ-,
- (CH₂-CH₂-O)ₙ-(CH₂-CH(CH₃)-O)ₒ-(CH₂-CH₂-O)ₚ-,
- (CH₂-CH₂-O)ₙ-(CH(CH₃)-CH₂-O)ₒ-(CH₂-CH₂-O)ₚ-,
- (CH₂-CH₂-O)ₙ-(CH(CH₃)CH₂-O)ₒ-,
- (CH₂-CH₂-O)ₙ-(CH₂-CH(CH₃)-O)ₒ-,
- (CH₂-CH(CH₃)-O)ₒ-(CH₂-CH₂-O)ₙ-,
- (CH(CH₃)-CH₂-O)ₒ-(CH₂-CH₂-O)ₙ-,
- (CH₂-CH(CH₃)-O)ₒ-(CH₂-CH₂-O)ₙ-(CH₂-CH(CH₃)-O)_{q}-,
- (CH₂-CH(CH₃)-O)ₒ-(CH₂-CH₂-O)ₙ-(CH(CH₃)-CH₂-O)_{q}-,
- (CH(CH₃)-CH₂O)ₒ-(CH₂-CH₂-O)ₙ-(CH₂-CH(CH₃)-O)_{q}- et
- (CH(CH₃)-CH₂O)ₒ-(CH₂-CH₂-O)ₙ-(CH(CH₃)-CH₂-O)ₒ-,
R⁶ est un atome d'hydrogène ou un groupe alkyle en C₁₋₄ ; et
R⁷ est un groupe alkyle en C₁₋₄
m est un nombre entier allant de 1 à 4
n et p sont des nombres entiers compris entre 1 et 10
m et o sont des nombres entiers compris entre 1 et 10
et des sels et solvates cosmétiquement acceptables de ceux-ci, à la surface externe de la microcapsule après formation de la microcapsule.

10. Procédé selon la revendication 9, où la microcapsule est une microcapsule à double couche polymère ayant un polymère de couche interne et un polymère de couche externe et l'étape a) consistant à former la microcapsule comprend les étapes consistant à
a1) mélanger le polymère de couche interne avec le composé antivieillissement dans un solvant approprié,
a2) émulsifier le mélange obtenu à l'étape a) avec le polymère de couche externe dans un solvant approprié, et facultativement
a3) isoler les microcapsules.

11. Composition cosmétique comprenant la microcapsule telle que définie dans l'une quelconque des revendications 2 à 10.

12. Utilisation cosmétique d'un peptide tel que défini dans la revendication 1 pour favoriser la synthèse de collagène de type I et ou la synthèse d'élastine.

13. Utilisation des microcapsules telles que définies dans l'une quelconque des revendications 2 à 10 pour la prévention cosmétique et/ou le traitement cosmétique du vieillissement de la peau.

14. Utilisation des microcapsules telles que définies dans l'une quelconque des revendications 2 à 10, afin d'améliorer l'aspect de la peau par la réalisation d'un ou de plusieurs des effets suivants : augmentation de la luminosité de la peau, augmentation de l'élasticité de la peau, diminution de la rugosité de la peau, réduction des rides de la peau, augmentation de l'amincissement apparent de la peau, réduction des défauts de pigmentation de la peau.


### SEQUENCE LISTING

<110> Infinitec Activos, S.L. Peptidepharma Nova, S.L.
<120> Targeted capsules to fibroblast for the delivery of antiaging agents in the skin
<130> P11593PC00
<150> EP15382077.4
   <151> 2015-02-25
<160> 13
<170> PatentIn version 3.5
<210> 1
   <211> 12
   <212> PRT
   <213> Artificial sequence
<220>
   <223> Peptide 1
<400> 1
   Lys Phe Asn Leu Pro Leu Gly Asn Tyr Lys Lys Pro
   1               5                   10
<210> 2
   <211> 12
   <212> PRT
   <213> Artificial sequence
<220>
   <223> Peptide 2
<400> 2
   Lys Ala Asn Leu Pro Leu Gly Asn Tyr Lys Lys Pro
   1               5                   10
<210> 3
   <211> 12
   <212> PRT
   <213> Artificial sequence
<220>
   <223> Peptide 3
<400> 3
   Lys Phe Ala Leu Pro Leu Gly Asn Tyr Lys Lys Pro
   1               5                   10
<210> 4
   <211> 12
   <212> PRT
   <213> Artificial sequence
<220>
   <223> Peptide 4
<400> 4
   Lys Phe Asn Ala Pro Leu Gly Asn Tyr Lys Lys Pro
   1               5                   10
<210> 5
   <211> 12
   <212> PRT
   <213> Artificial sequence
<220>
   <223> Peptide 5
<400> 5
   Lys Phe Asn Leu Ala Leu Gly Asn Tyr Lys Lys Pro
   1               5                   10
<210> 6
   <211> 12
   <212> PRT
   <213> Artificial sequence
<220>
   <223> Peptide 6
<400> 6
   Lys Phe Asn Leu Pro Ala Gly Asn Tyr Lys Lys Pro
   1               5                   10
<210> 7
   <211> 12
   <212> PRT
   <213> Artificial sequence
<220>
   <223> Peptide 7
<400> 7
   Lys Phe Asn Leu Pro Leu Ala Asn Tyr Lys Lys Pro
   1               5                   10
<210> 8
   <211> 12
   <212> PRT
   <213> Artificial sequence
<220>
   <223> Peptide 8
<400> 8
   Lys Phe Asn Leu Pro Leu Gly Ala Tyr Lys Lys Pro
   1 5 10
<210> 9
   <211> 12
   <212> PRT
   <213> Artificial sequence
<220>
   <223> Peptide 9
<400> 9
   Lys Phe Asn Leu Pro Leu Gly Asn Ala Lys Lys Pro
   1 5 10
<210> 10
   <211> 12
   <212> PRT
   <213> Artificial sequence
<220>
   <223> Peptide 10
<400> 10
   Lys Phe Asn Leu Pro Leu Gly Asn Tyr Ala Lys Pro
   1 5 10
<210> 11
   <211> 12
   <212> PRT
   <213> Artificial sequence
<220>
   <223> Peptide 11
<400> 11
   Lys Phe Asn Leu Pro Leu Gly Asn Tyr Lys Ala Pro
   1 5 10
<210> 12
   <211> 12
   <212> PRT
   <213> Artificial sequence
<220>
   <223> Peptide 12
<400> 12
   Lys Phe Asn Leu Pro Leu Gly Asn Tyr Lys Lys Ala
   1 5 10
<210> 13
   <211> 18
   <212> PRT
   <213> Artificial Sequence
<220>
   <223> Substrate
<400> 13
   Cys Ala Gly Ala Gly Ala Ile Glu Thr Asp Lys Glu Tyr Tyr Thr Val
   1               5                   10                  15
   Lys Asp